# EUROPEAN PATENT APPLICATION

(11) **EP 2 974 654 A1**
(43) Date of publication of application: **20.01.2016**
(21) Application number: 14765378.6
(22) Date of filing: 14.03.2014
(51) Int. Cl.: A61B 5/12

(54) **HEARING EXAMINATION DEVICE, HEARING EXAMINATION METHOD, AND METHOD FOR GENERATING WORDS FOR HEARING EXAMINATION**

(30) Priority: 15.03.2013 JP 2013054053
(71) Applicant: Nitto Denko Corporation, Ibaraki-shi, Osaka 567-8680 (JP)
(72) Inventor: MORITA, Shigenori, Ibaraki-shi Osaka 567-8680 (JP); HAYAMA, Hideki, Ibaraki-shi Osaka 567-8680 (JP); BAN, Naoki, Ibaraki-shi Osaka 567-8680 (JP); MIMOTO, Akiko, Ibaraki-shi Osaka 567-8680 (JP); MATSUSHIMA, Ryouichi, Ibaraki-shi Osaka 567-8680 (JP); MASAKI, Toshiaki, Ibaraki-shi Osaka 567-8680 (JP); KUSUURA, Takahisa, Yokohama-shi Kanagawa 225-0011 (JP)
(74) Representative: Müller-Boré & Partner Patentanwälte PartG mbB
(86) International application number: PCT/JP2014/056994
(87) International publication number: WO 2014/142328

(57) **Abstract**

The device of the present invention contains a recording unit (2) on which a plurality of words having possibilities of being misheard are recorded in a state of being classified on the basis of highness and lowness of the possibilities, a question output unit (4) that outputs, by voice sound, a question sentence containing at least one of the words having possibilities of being misheard, an input unit (5) that receives input of a response to the question sentence, a determining unit (12) that determines at least whether the hearing ability of a respondent is decreased or not on the basis of the content of the response input into the input unit (5), and a result output unit (6) that outputs a determination result determined by the determining unit (12).

## Description

### TECHNICAL FIELD

The present invention relates to a hearing test device, hearing test method and hearing test word creating method.

### BACKGROUND ART

A hearing aid is an important accessory for a hearing-impaired person to live a comfortable life. When a person does not wear a hearing aid despite the necessity of wearing a hearing aid, symptoms of hardness of hearing progress, and the person may lose language recognition ability when the symptoms of hardness of hearing progress too far. Even if the person uses a hearing aid after being in such a state, the person may not understand words although being capable of recognizing sound. Therefore, it is important to start wearing a hearing aid at an appropriate time.

Regarding this matter, hearing aid makers and stores provide various opportunities so as to be capable of providing each person hard of hearing with an appropriate hearing aid early. For example, hearing consultations are held for people who worry about the hearing ability, and simple hearing diagnostic tests are provided on websites so as to be used freely. In addition, for example, there is suggested a hearing aid sales support system that uses the Internet (refer to Patent Document 1).

### BACKGROUND ART DOCUMENTS

### PATENT DOCUMENTS

Patent Document 1: JP-A-2002-259714

### SUMMARY OF INVENTION

### PROBLEMS THAT INVENTION IS TO SOLVE

When a person is aware of hardness of hearing with his/her ears in daily life, the person can go to an otological clinic to receive a diagnosis or can visit a hearing aid store to have a consultation and to receive a hearing test. However, since decrease in hearing ability progresses gradually, it is difficult to realize in an initial state. In addition, when an opportunity of receiving a hearing test is set by using a system such as disclosed in Patent Document 1, a person who is unaware of weakened hearing ability may feel resistance in receiving a hearing test. In this case, even though a hearing test is recommended to a person who is unaware of weakened hearing ability, this may impair the self-esteem of the person, and the person may not receive a hearing test.

An object of the present invention is to provide a hearing test device, a hearing test method, and a hearing test word creating method, in which a person who is unaware of weakened hearing ability can receive a hearing test in a natural way without giving uncomfortable feelings.

### MEANS FOR SOLVING PROBLEMS

In order to solve the above problem, the hearing test device according to the present invention contains:
a recording unit on which a plurality of words having possibilities of being misheard are recorded in a state of being classified on the basis of highness and lowness of the possibilities;
a question output unit that outputs a question sentence containing at least one of the words by voice sound;
an input unit that receives input of a response to the question sentence;
a determining unit that determines at least whether the hearing ability of a respondent is decreased or not on the basis of the content of the response input into the input unit; and
a result output unit that outputs a determination result determined by the determining unit.

In the hearing test device according to the present invention, it is preferable that, on the recording unit, the word having a possibility of being misheard is recorded in a state of being linked to a candidate word of the mishearing thereof.

In the hearing test device according to the present invention, it is preferable that the question output unit outputs in order a plurality of question sentences that include the words having a different possibility of being misheard on the basis of the content of the response that is input into the input unit, and the determining unit determines a level of decreased hearing ability on the basis of the contents of the responses to the plurality of question sentences.

The hearing test method according to the present invention contains:
a question outputting step of outputting by voice sound a question sentence that includes at least one word selected from a plurality of words having different possibilities of being misheard;
an inputting step of receiving input of a response to the question sentence;
a determining step of determining at least whether the hearing ability of a respondent is decreased or not on the basis of the content of the response that is input through the inputting step; and
a result outputting step of outputting a determination result determined through the determining step.

The hearing test word creating method according to the present invention contains:
a word selecting step of selecting a word arbitrarily from a dictionary;
an altered hearing converting step of performing an altered hearing conversion on the word that is selected in the word selecting step;
a word searching step of searching for whether a word resulted from the altered hearing conversion in the altered hearing converting step exists on the dictionary; and
a word recording step of, when the word gets a hit as an existing word in the word searching step, recording the word on the recording unit.

In the hearing test word creating method according to the present invention, it is preferable that the altered hearing converting step is a process of replacing a part of the characters of the word that is selected in the word selecting step on the basis of an altered hearing matrix that indicates altered hearing tendencies of a predetermined word group.

In the hearing test word creating method according to the present invention, it is preferable that when there are a plurality of words that get hits as existing words in the word searching step, they are compared with information recorded in a database on a network, and in consequence of the comparison, a word having a high frequency of use is recorded on the recording unit in the word recording step.

### ADVANTAGEOUS EFFECTS OF INVENTION

According to the present invention, it is possible to allow a person who is unaware of weakened hearing ability to receive a hearing test in a natural way without giving uncomfortable feelings.

### BRIEF DESCRIPTION OF DRAWINGS

[FIG. 1] It is a diagram illustrating a configuration of a hearing test device according to the present invention.
[FIG. 2] It is a diagram illustrating an example of a word group that is recorded on a recording unit illustrated in FIG. 1.
[FIG. 3] It is a flowchart illustrating an example of a hearing test procedure according to the present invention.
[FIG. 4] It is a flowchart illustrating an example of the hearing test procedure according to the present invention.
[FIG. 5] It is a flowchart illustrating an example of the hearing test procedure according to the present invention.
[FIG. 6] It is a flowchart illustrating an example of the hearing test procedure according to the present invention.
[FIG. 7] It is a flowchart illustrating an example of a hearing test word creating procedure according to the present invention.

### MODES FOR CARRYING OUT THE INVENTION

Hereinafter, an example of embodiments of a hearing test device, a hearing test method and a hearing test word creating method according to the present invention will be described on the basis of the appended drawings. While the present invention is not limited to Japanese or English and is able to be applied to other languages in the same manner, the example below will be described in the case where Japanese is used. In the description below, a word in square brackets ┌┘ indicates a Japanese word (hiragana, katakana, or kanji), and a representation in parentheses () between square brackets ┌┘ is a Roman letter representation of the pronunciation (sound) of the Japanese word in the square brackets ┌┘. For example, " 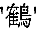 " of ┌ 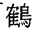 (TURU)┘ is a Japanese word, and "TURU" is the Roman letter representation of the pronunciation (sound) of the Japanese word " 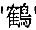 ".

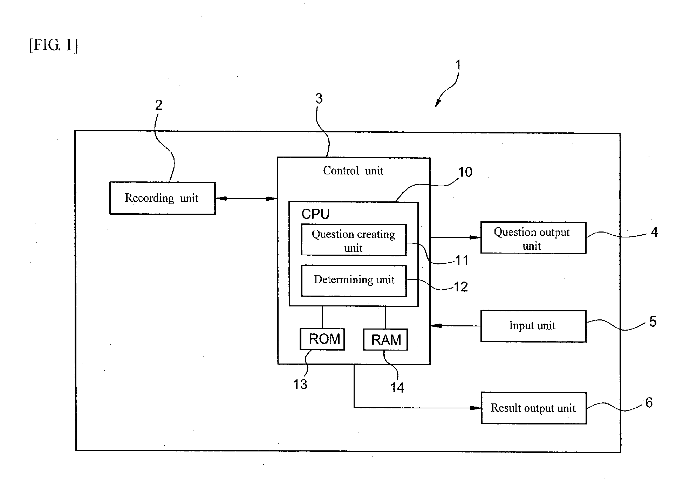

FIG. 1 illustrates a configuration of a hearing test device 1. As illustrated in FIG. 1, the hearing test device 1 is provided with a recording unit 2 on which a word group used in a hearing test is recorded, a control unit 3 that controls an operation of a hearing test device 1, a question output unit 4 that outputs a test question sentence used in a hearing test, an input unit 5 for inputting a response to a question in a hearing test, and a result output unit 6 that outputs a determination result of a hearing test.

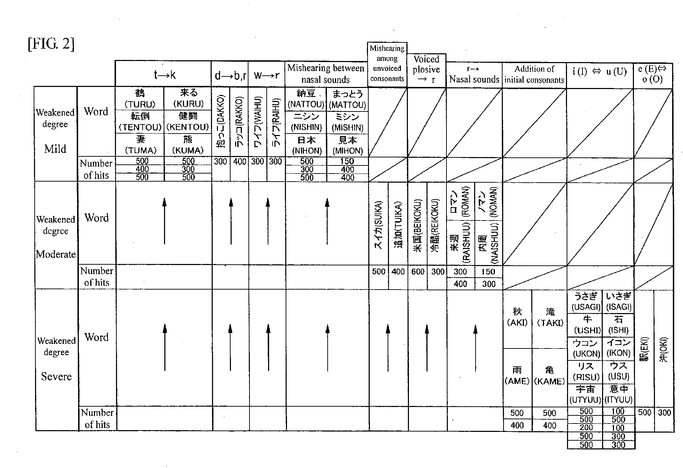

The recording unit 2 records words that a person having weakened hearing ability may mishear. The recording unit 2 is configured of, for example, a database, and on which a plurality of words that may be misheard are recorded so as to be linked to corresponding candidate words of the mishearing thereof. The words will be specifically described below with reference to FIG. 2.

The control unit 3 controls an operation of each unit of the device 1 at the time of a hearing test. The control unit 3 includes a CPU 10 as a main constituent thereof. The CPU 10 performs various processes in accordance with a program stored on a ROM 13 and uses a RAM 14 as an area for storing various data.

The CPU 10 is provided with a question creating unit 11 and a determining unit 12. The CPU 10 has a question creating function as the question creating unit 11 and has a hearing ability determining function as the determining unit 12.

The question creating unit 11 arbitrarily selects word(s) recorded on the recording unit 2 which may be misheard and creates a question sentence for a hearing test that includes at least one of the selected word(s). Example sentences constituting a question sentence are prepared in advance and, for example, are stored on an unillustrated storing unit. The question creating unit 11 creates a question sentence by combining a word selected from the recording unit 2 and an example sentence stored on the storing unit. An example sentence that is combined with a selected word may be, for example, recorded in advance on the recording unit 2 or may be created by an operator with the input unit 5.

A question sentence created by the question creating unit 11 is output through the question output unit 4. The question output unit 4 outputs a question sentence by voice sound and presents the output question sentence to a respondent, that is, to a subject of a hearing test. The question output unit 4 can be configured of, for example, a speaker. It may be configured in the form in which a tester who performs a hearing test reads a question sentence to a respondent (subject).

The content of a question sentence that is output by voice sound from the question creating unit 11 can be presented to a respondent, for example, in the form of a questionnaire related to a certain theme or in the form of a game.

A response of a respondent to a question sentence output (presented) from the question output unit 4 is input (responded) through the input unit 5. The input unit 5 can be configured of, for example, a microphone into which a response of a respondent is input as a voice. The input unit 5 may also be configured of a keyboard or a touch panel into which a response of a respondent is input in characters. A response that is input into a microphone and the like 5 is converted into an electrical signal and is input into the control unit 3.

A response that is input from the input unit 5 is input into the determining unit 12 of the control unit 3. The determining unit 12 determines whether the hearing ability of a respondent is decreased and further determines the level of decreased hearing ability of the respondent on the basis of the content of an input response. A determination of whether the hearing ability of a respondent is decreased is performed by determining whether he/she can accurately listen to a question sentence, that is, by determining whether he/she does.not mishear a word included in a question sentence which may be misheard. The level of decreased hearing ability, when a respondent mishears a word that may be misheard, is determined by the type (altered hearing ability tendency) of character that is misheard. The level of hearing ability will be specifically described below with reference to FIG. 2.

The result of a hearing test determined by the determining unit 12 is output through the result output unit 6. The result output unit 6 can be configured of, for example, a display screen (an LCD, an organic EL, etc.). Displayed on the result output unit 6 are determination results of a hearing test, that is, a determination of whether hearing ability is decreased, the level of a decrease when decreased hearing ability is detected, and the like. The result output unit 6 may be configured to include a print unit so that a determination result can be printed out.

FIG. 2 illustrates an example of an altered hearing matrix that indicates a word group recorded on the recording unit 2 which may be misheard. As displayed in FIG. 2, the degree of weakened hearing ability is classified into three stages of a mild degree, a moderate degree, and a severe degree, and the degrees are displayed therein in this order in the upper row, the middle row, and the lower row. Therein, words that may be misheard in the corresponding weakened degree are displayed so as to be linked to the corresponding candidate words of the mishearing thereof alongside in each row. FIG. 2 illustrates an altered hearing tendency depending on the degree of weakened hearing ability.

In the case of the mild degree of weakened hearing ability displayed in the upper row, there are a tendency to mishear "t" as "k", a tendency to mishear "d" as "b" or "r", a tendency to mishear "w" as "r", and a tendency to mishear between nasal sounds ("n" and "m").

Examples of mishearing "t" as "k" can include examples of mishearing ┌ 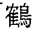 (TURU)J as ┌ 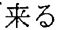 (KURU)┘, ┌ 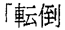 (TENTOU)┘ as ┌ 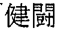 (KENTOU)┘, and ┌ 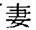 (TUMA)┘ as ┌ 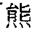 (KUMA)┘.

Examples of mishearing "d" as "b" or "r" can include an example of mishearing ┌ 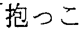 (DAKKO)┘ as ┌ 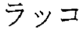 (RAKKO)┘.

Examples of mishearing "w" as "r" can include an example of mishearing ┌ 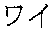 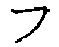 (WAIHU)┘ as ┌ 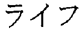 (RAIHU)┘.

Examples of mishearing between nasal sounds ("n" as "m") can include examples of mishearing ┌ 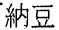 (NATTOU)┘ as ┌ 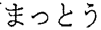 (MATTOU)┘, ┌ 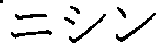 (NISHIN)J as ┌ 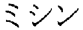 (MISHIN)┘, and ┌ 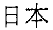 (NIHON)┘ as ┌ 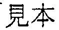 (MIHON)┘.

In the case of the moderate degree of weakened hearing ability displayed in the middle row, in addition to the case of the mild degree of weakened hearing ability, there are a tendency to mishear among unvoiced consonants, a tendency to mishear a "voiced plosive" as "r", and a tendency to mishear "r" as a "nasal sound".

Examples of the tendency to mishear among unvoiced consonants can include an example of mishearing ┌ 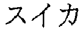 (SUIKA)┘ as ┌ 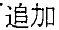 (TUIKA)┘.

Examples of mishearing a "voiced plosive" as "r" can include an example of mishearing ┌ 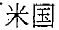 (BEIKOKU)┘ as ┌ 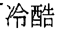 (REIKOKU)┘.

Examples of mishearing "r" as a "nasal sound" can include examples of mishearing ┌ 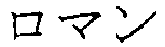 (ROMAN)J as ┌ 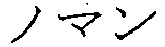 (NOMAN)┘ and ┌ 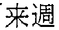 (RAISHUU)┘ as ┌ 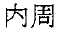 (NAISHUU)┘.

In the case of the severe degree of weakened hearing ability displayed in the lower row, in addition to the case of the moderate degree of weakened hearing ability, there are a tendency to addition of initial consonants, a tendency to mishear "i (I)" and "u (U)", and a tendency to mishear "e (E)" and "o (O)".

Examples of a tendency to addition of initial consonants can include examples of mishearing ┌ 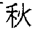 (AKI)┘ as ┌ 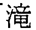 (TAKI)┘ and ┌ 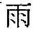 (AME)┘ as ┌ 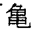 (KAME) ┘.

Examples of a tendency to mishear "i (I)" and "u (U)" can include examples of mishearing ┌ 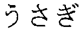 (USAGI)J and ┌ 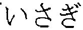 (ISAGI)┘, ┌ 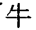 (USHI)┘ and ┌ 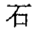 (ISHI)┘, ┌ 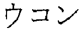 (UKON)┘ and ┌ 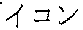 (IKON)┘, ┌ 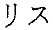 (RISU)] and ┌ 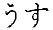 (USU)┘, and ┌ 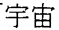 (UTYUU)J and ┌ 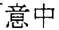 (ITYUU)┘.

Examples of a tendency to mishear "e (E)" and "o (O)" can include an example of mishearing ┌ 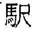 (EKI)┘ and ┌ 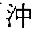 (OKI)┘.

Frequency information that indicates the frequency of the use of a word is stored by being attached to each stored word. For example, in FIG. 2, frequency information of 500 is attached to the word ┌ 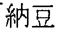 (NATTOU)J displayed in the upper row, frequency information of 150 is attached to ┌ 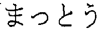 (MATTOU)J , 300 to ┌ 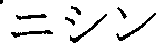 (NISHIN)┘, 400 to ┌ 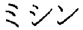 (MISHIN)┘, 500 to ┌ 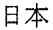 (NIHON)┘, and 400 to ┌ 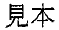 (MIHON)┘. The frequency information in FIG. 2 indicates the number of hits for each word searched on the Internet.

A word is arbitrarily selected from such a word group recorded on the recording unit 2, and a question sentence for a hearing test is created.

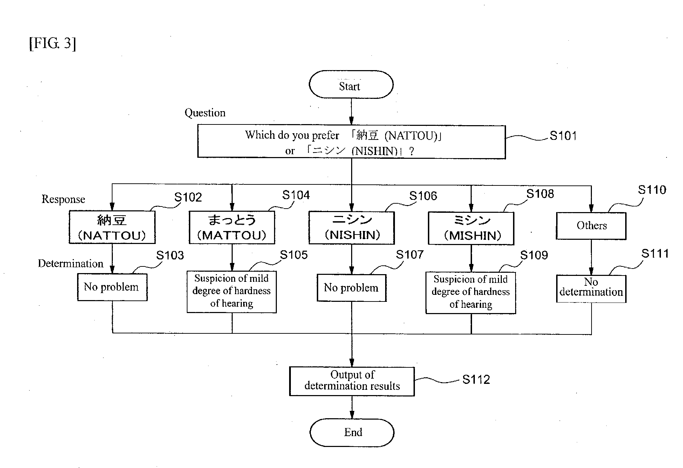

Next, a hearing test procedure will be described on the basis of the flowcharts illustrated in FIG. 3 to FIG. 6. Each of FIG. 3 to FIG. 6 illustrates an example of a hearing test pattern.

First, a tester speaks to a pedestrian on the street and asks him/her to receive a hearing test. In this case, the tester speaks to the pedestrian as a conductor who performs a questionnaire so as not to be noticed by the pedestrian that it is a test for testing hearing ability. Then, the hearing ability of the respondent (pedestrian) is tested in the form of responding to the questionnaire. In the case of speaking to a pedestrian, considering an age group in which weakened hearing ability is generally likely to occur is desired without selecting randomly.

FIG. 3 is a hearing test pattern of a method in which one word is selected from two or more words.

When a hearing test starts, word(s) are selected from the recording unit 2 by the question creating unit 11 of the hearing test device 1. A question sentence that includes at least one of the word(s) is created and is output toward the respondent from the question output unit 4. That is, the question output unit 4 outputs the question sentence "Which do you prefer ┌ 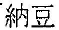 (NATTOU)J or ┌ 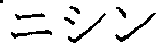 (NISHIN)┘ ?" by voice sound to the respondent (step S101, an example of a question outputting step). The words ┌ 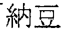 (NATTOU)J and ┌ 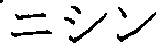 (NISHIN)┘ included in the question sentence are words that are likely to be misheard when weakened hearing ability occurs and are one in the word group that is recorded in advance on the recording unit 2 (refer to FIG. 2).

A response of the respondent to the question is, for example, input into the hearing test device 1 through the input unit 5 (an example of an inputting step). When the respondent responds to the question with ┌ 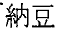 (NATTOU)┘ (step S102), the respondent responds to the word included in the question sentence and does not mishear the word ┌ 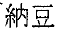 (NATTOU)┘. As displayed in the table of altered hearing tendencies in FIG. 2, ┌ 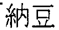 (NATTOU)J is classified as a word that is likely to be misheard by a person who has a mild degree of weakened hearing ability. Therefore, determination by the determining unit 12 in this hearing test pattern yields a determination result of "having no problem in hearing ability" (step S 103, an example of a determining step).

When the respondent responds to the question with ┌ 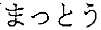 (MATTOU)J (step S104), it is determined that the respondent mishears ┌ 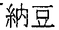 (NATTOU)┘ as ┌ 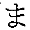 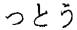 (MATTOU)J . As displayed in the table of altered hearing tendencies in FIG. 2, altered hearing between nasal sounds such as ┌ 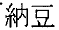 (NATTOU)┘ and ┌ 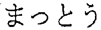 (MATTOU)┘ is displayed in the upper row, the middle row, and the lower row as words that are likely to be misheard by a person who has a mild degree, a moderate degree, or a severe degree of weakened hearing ability. Therefore, in this case, determination in this hearing test pattern yields a determination result of "suspected to have a mild degree of hardness of hearing (also possibly a moderate degree or a severe degree)" (step S105).

When the respondent responds to the question with ┌ 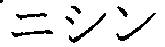 (NISHIN)┘ (step S106), the respondent does not mishear the word ┌ 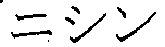 (NISHIN)┘. As displayed in the table of altered hearing tendencies in FIG. 2, ┌ 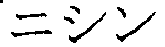 (NISHIN)┘ is classified as a word that is likely to be misheard by a person who has a mild degree of weakened hearing ability. Therefore, determination in this hearing test pattern yields a determination result of "having no problem in hearing ability" (step S107).

When the respondent responds to the question with ┌ 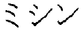 (MISHIN)┘ (step S108), it is determined that the respondent mishears ┌ 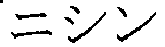 (NISHIN)┘as ┌ 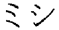 (MISHIN)J . As displayed in the table of altered hearing tendencies in FIG. 2, altered hearing between nasal sounds such as ┌ 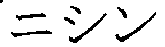 (NISHIN)┘ and ┌ 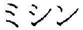 (MISHIN)┘ is displayed in the upper row, the middle row, and the lower row as words that are likely to be misheard by a person who has a mild degree, a moderate degree, or a severe degree of weakened hearing ability. Therefore, in this case, determination in this hearing test pattern yields a determination result of "suspected to have a mild degree of hardness of hearing (also possibly a moderate degree or a severe degree)" (step S109).

When the respondent responds to the question with a word other than ┌ 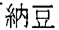 (NATTOU)┘, ┌ 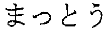 (MATTOU)┘, ┌ 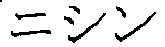 (NISHIN)┘, or ┌ 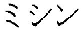 (MISHIN)┘ (step S110), determination in this hearing test pattern yields a result of "not determined" (step S111).

These determination results are output to the respondent through the result output unit 6 (step S112, an example of a result outputting step).

The determination results are recorded on the recording unit 2 as customer hearing ability information along with name, address and the like of the respondent.

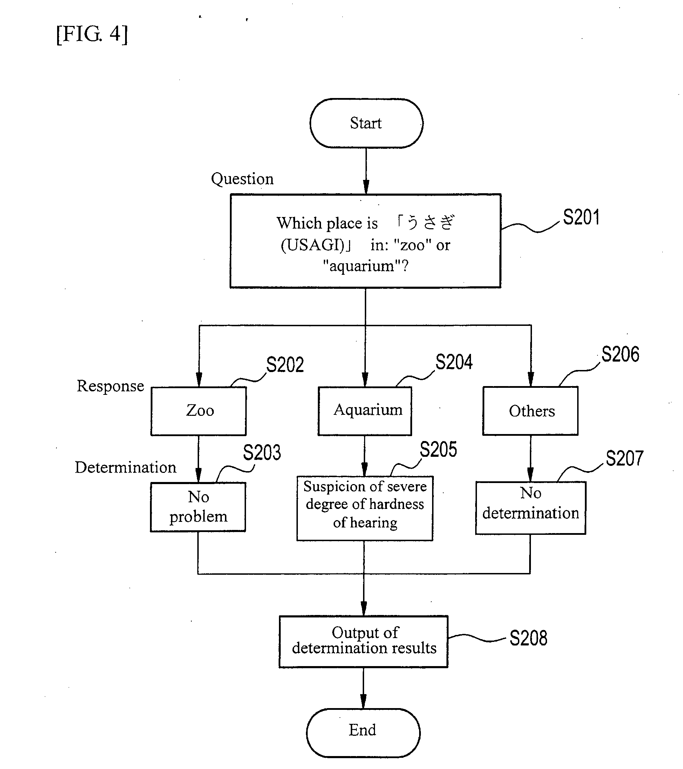

Next, FIG. 4 is a hearing test pattern of a method in which a word that is correlated with one word is selected from the one word.

When a hearing test starts, a question sentence is created by the question creating unit 11, and the created question sentence "Which place is ┌ 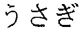 (USAGI)J in: "zoo" or "aquarium"?" is, for example, output by voice sound and is presented to the respondent through the question output unit 4 (step S201, an example of a question outputting step). The word ┌ 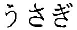 (USAGI)┘ included in the question sentence is a word that is likely to be misheard by a person who has weakened hearing ability and is one in the word group that is recorded in advance on the recording unit 2 (refer to FIG. 2).

A response of the respondent to the question is, for example, input through the input unit 5 (an example of an inputting step). When the respondent responds to the question with "zoo" (step S202), the respondent does not mishear the word ┌ 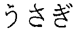 (USAGI)┘. However, as displayed in the table of altered hearing tendencies in FIG. 2, ┌ 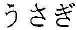 (USAGI)┘ is classified in the lower row as a word that is likely to be misheard by a person who has a severe degree of weakened hearing ability. Therefore, determination by the determining unit 12 in this hearing test pattern yields a determination result of "not corresponding to a severe degree of weakness (possibly having a moderate degree or a mild degree of weakness)" (step S203, an example of a determining step).

When the respondent responds to the question with "aquarium" (step S204), it is determined that the respondent mishears ┌ 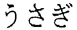 (USAGI)J as ┌ 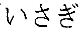 (ISAGI)┘ . As displayed in the table of altered hearing tendencies in FIG. 2, altered hearing between "i (I)" and "u (U)" such as ┌ 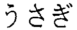 (USAGI)┘ and ┌ 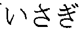 (ISAGI)J is displayed in the lower row as words that are likely to be misheard by a person who has a severe degree of weakened hearing ability. Therefore, in this case, determination in this hearing test pattern yields a determination result of "suspected to have a severe degree of hardness of hearing" (step S205).

When the respondent responds to the question with a word other than "zoo" or "aquarium" (step S206), determination in this hearing test pattern yields a result of "not determined" (step S207).

These determination results are output to the respondent by the result output unit 6 (step S208). Recording the determination results on the recording unit 2 is the same as that in the hearing test pattern illustrated in FIG. 3.

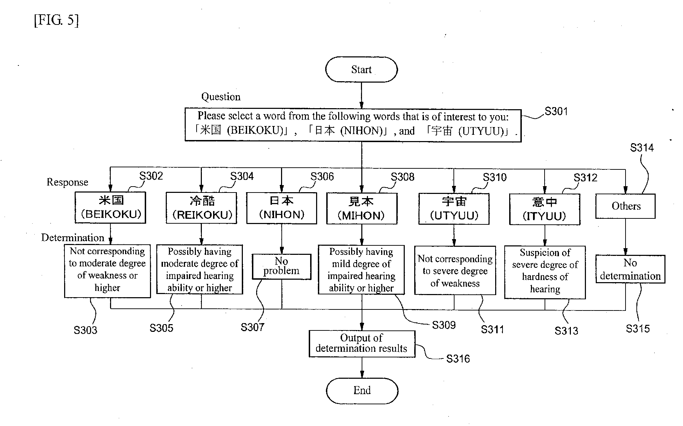

Next, FIG. 5 is an example of a hearing test pattern of a method in which one word is selected from among three words.

When a hearing test starts, a question sentence is created by the question creating unit 11, and the created question sentence "Please select a word from the following words that is of interest to you: ┌ 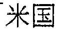 (BEIKOKU)┘, ┌ 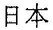 (NIHON)┘, and ┌ 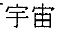 (UTYUU)┘ ." is, for example, output to the respondent from the question output unit 4 (step S301, an example of a question outputting step). All of the words ┌ 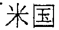 (BEIKOKU)┘, ┌ 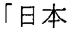 (NIHON)┘, and ┌ 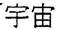 (UTYUU)┘ included in the question sentence are words that are likely to be misheard by a person who has weakened hearing ability and are one in the word group that is recorded in advance on the recording unit 2 (refer to FIG. 2).

A response of the respondent to the question is, for example, input through the input unit 5 (an example of an inputting step). When the respondent responds to the question with ┌ 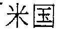 (BEIKOKU)┘ (step S302), the respondent does not mishear the word ┌ 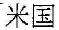 (BEIKOKU)┘. However, as displayed in the table of altered hearing tendencies in FIG. 2, ┌ 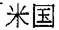 (BEIKOKU)┘ is classified as a word that is likely to be misheard by a person who has a moderate degree or a severe degree of weakened hearing ability. Therefore, determination in this hearing test pattern yields a determination result of "not corresponding to a moderate degree of weakness or higher (having no problem, or possibly having a mild degree of weakness)" (step S303, an example of a determining step).

When the respondent responds to the question with ┌ 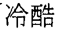 (REIKOKU)J (step S304), it is determined that the respondent mishears ┌ 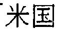 (BEIKOKU)┘ as ┌ 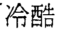 (REIKOKU)┘. As displayed in the table of altered hearing tendencies in FIG. 2, altered hearing of "voiced plosive" and "r" such as ┌ 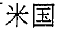 (BEIKOKU)┘ and ┌ 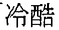 (REIKOKU)J is displayed in the middle row and in the lower row as words that are likely to be misheard by a person who has a moderate degree and a severe degree of weakened hearing ability. Therefore, in this case, determination in this hearing test pattern yields a determination result of "possibly having a moderate degree of impaired hearing ability or higher" (step S305).

When the respondent responds to the question with ┌ 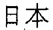 (NIHON)┘ (step S306), the respondent does not mishear the word ┌ 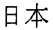 (NIHON)┘. As displayed in the table of altered hearing tendencies in FIG. 2, ┌ 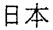 (NIHON)┘ is classified as a word that is likely to be misheard by a person who has a mild degree of weakened hearing ability. Therefore, determination in this hearing test pattern yields a determination result of "having no problem in hearing ability" (step S307).

When the respondent responds to the question with ┌ 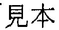 (MIHON)┘ (step S308), it is determined that the respondent mishears ┌ 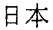 (NIHON)┘ as ┌ 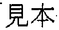 (MIHON)J . As displayed in the table of altered hearing tendencies in FIG. 2, altered hearing among nasal sounds such as ┌ 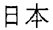 (NIHON)┘ and ┌ 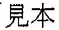 (MIHON)J is displayed in the upper row, the middle row, and the lower row as words that are likely to be misheard by a person who has a mild degree, a moderate degree, or a severe degree of weakened hearing ability. Therefore, in this case, determination in this hearing test pattern yields a determination result of "possibly having a mild degree of impaired hearing ability or higher" (step S309).

When the respondent responds to the question with ┌ 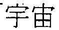 (UTYUU)J (step S310), the respondent does not mishear the word ┌ 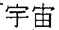 (UTYUU)J . However, as displayed in the table of altered hearing tendencies in FIG. 2, ┌ 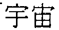 (UTYUU)┘ is classified in the lower row as a word that is likely to be misheard by a person who has a severe degree of weakened hearing ability. Therefore, determination in this hearing test pattern yields a determination result of "not corresponding to a severe degree of weakness (possibly having a moderate degree or a mild degree of weakness)" (step S311).

When the respondent responds to the question with ┌ 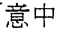 (ITYUU)┘ (step S312), it is determined that the respondent mishears ┌ 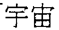 (UTYUU)┘ as ┌ 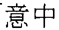 (ITYUU)┘. As displayed in the table of altered hearing tendencies in FIG. 2, altered hearing between "i (I)" and "u (U)" such as ┌ 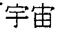 (UTYUU)┘ and ┌ 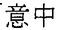 (ITYUU)J is displayed in the lower row as words that are likely to be misheard by a person who has a severe degree of weakened hearing ability. Therefore, in this case, determination in this hearing test pattern yields a determination result of "suspected to have a severe degree of hardness of hearing" (step S313).

When the respondent responds to the question with a word other than ┌ 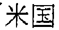 (BEIKOKU)┘, ┌ 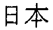 (NIHON)┘, or ┌ 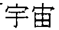 (UTYUU)┘ (step S314), determination in this hearing test pattern yields a result of "not determined" (step S315).

These determination results are output to the respondent through the result output unit 6 (step S316). Storing the determination results on the recording unit 2 is the same as that in the hearing test pattern illustrated in FIG. 3.

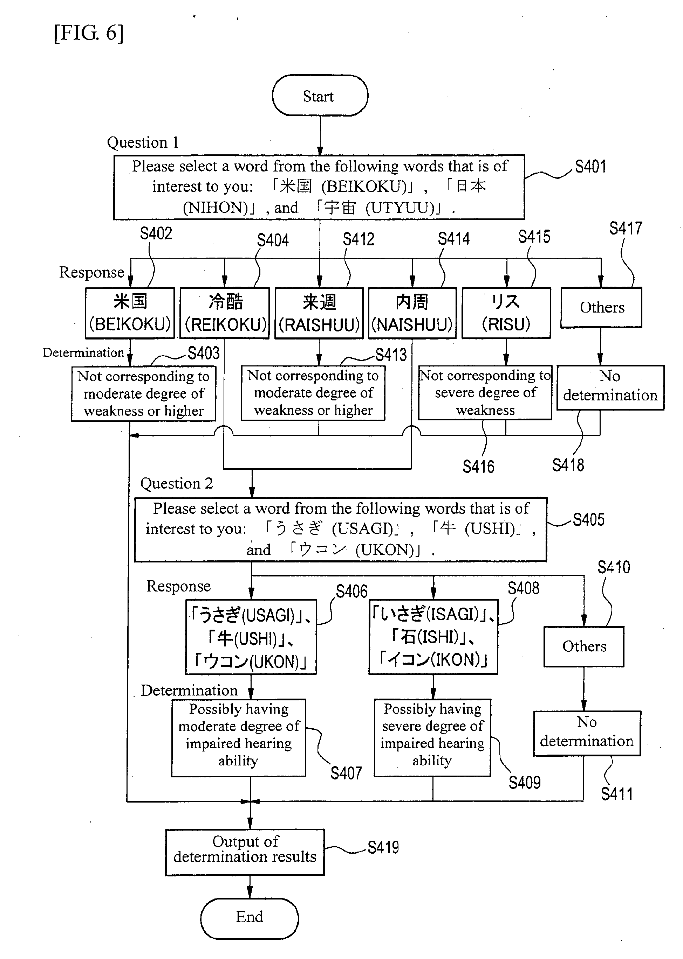

Next, FIG. 6 is a modification example of the hearing test pattern of the method in which one word is selected from two or more words. In this modification example, a hearing test is performed by presenting a plurality of question sentences in order in response to a response of the respondent.

When a hearing test starts, a question sentence is created by the question creating unit 11, and the created question sentence "Please select a word from the following words that is of interest to you: ┌ 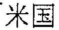 (BEIKOKU)┘, ┌ 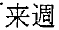 (RAISHUU)┘, and ┌ 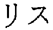 (RISU)J ." is, for example, output to the respondent from the question output unit 4 (step S401, an example of a question outputting step). All of the words ┌ 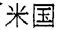 (BEIKOKU)┘, ┌ 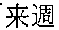 (RAISHUU)┘, and ┌ 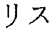 (RISU)┘ included in the question sentence are words that are likely to be misheard by a person who has weakened hearing ability and are one in the word group that is recorded in advance on the recording unit 2 (refer to FIG. 2).

A response of the respondent to the question is, for example, input through the input unit 5 (an example of an inputting step). When the respondent responds to the question (referred to as a first question) with ┌ 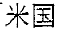 (BEIKOKU)┘ (step S402), the respondent does not mishear the word ┌ 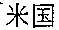 (BEIKOKU)┘. However, as displayed in the table of altered hearing tendencies in FIG. 2, ┌ 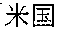 (BEIKOKU)┘ is classified as a word that is likely to be misheard by a person who has a moderate degree or a severe degree of weakened hearing ability. Therefore, determination in this hearing test pattern yields a determination result of "not corresponding to a moderate degree of weakness or higher (having no problem, or possibly having a mild degree of weakness)" (step S403, an example of a determining step).

When the respondent responds to the first question with ┌ 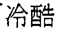 (REIKOKU)┘ (step S404), it is determined that the respondent mishears ┌ 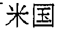 (BEIKOKU)┘as ┌ 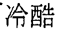 (REIKOKU)┘. In the same manner as described in steps S304 and S305 of FIG. 5, determination in this hearing test pattern yields a result of "possibly having a moderate degree of impaired hearing ability or higher". That is, the determination result indicates the possibility of having a moderate degree of impaired hearing ability and also indicates the possibility of having a severe degree thereof.

Then, in order to clarify the determination result, a second question sentence "Please select a word from the following words that is of interest to you: ┌ 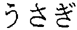 (USAGI)┘, ┌ 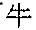 (USHI)┘, and ┌ 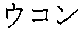 (UKON)┘." is next presented to the respondent (step S405).

When the respondent responds to the second question with ┌ 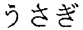 (USAGI)┘, ┌ 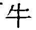 (USHI)J or ┌ 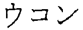 (UKON)J (step S406), the respondent does not mishear the words ┌ 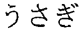 (USAGI)┘, ┌ 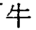 (USHI)┘, and ┌ 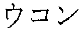 (UKON)┘. As displayed in the table of altered hearing tendencies in FIG. 2, ┌ 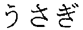 (USAGI)┘, ┌ 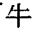 (USHI)┘ and ┌ 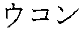 (UKON)J are classified in the lower row as words that are likely to be misheard by a person who has a severe degree of weakened hearing ability. Therefore, determination of hearing ability in the second question sentence yields a determination result of "not corresponding to a severe degree of weakness".

Thus, by outputting question sentences configured of the first and the second questions in order, the final determination in this hearing test pattern yields a determination result of "possibly having a moderate degree of impaired hearing ability" (step S407).

When the respondent responds to the second question with any one of ┌ 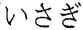 (ISAGI)J , ┌ 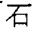 (ISHI)J and ┌ 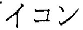 (IKON)J (step S408), it is determined that the respondent mishears ┌ 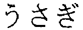 (USAGI)┘ as ┌ 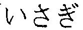 (ISAGI)┘, ┌ 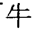 (USHI)J as ┌ 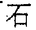 (ISHI)J or ┌ 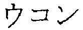 (UKON)┘ as ┌ 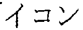 (IKON)J . As displayed in the table of altered hearing tendencies in FIG. 2, such an altered hearing of "i (I)" and "u (U)" is displayed in the lower row as words that are likely to be misheard by a person who has a severe degree of weakened hearing ability. Therefore, determination of hearing ability in the second question sentence yields a determination result of "possibly having a severe degree of impaired hearing ability".

Thus, by outputting question sentences of the first and the second questions in order, the final determination in this hearing test pattern yields a determination result of "possibly having a severe degree of impaired hearing ability" (step S409).

When the respondent responds to the second question with a word other than the above (step S410), determination in this hearing test pattern yields a result of "not determined" (step S411).

When the respondent responds to the first question with ┌ 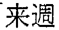 (RAISHUU)J (step S412), the respondent does not mishear the word ┌ 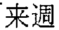 (RAISHUU)┘. However, as displayed in FIG. 2, ┌ 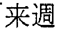 (RAISHUU)J is classified as a word that is likely to be misheard by a person who has a moderate degree or a severe degree of weakened hearing ability. Therefore, determination in this hearing test pattern yields a determination result of "not corresponding to a moderate degree of weakness or higher (having no problem, or possibly having a mild degree of weakness)" (step S413).

When the respondent responds to the first question with ┌ 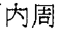 (NAISHUU)J (step S414), it is determined that the respondent mishears ┌ 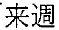 (RAISHUU)┘ as ┌ 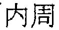 (NAISHUU)┘. As displayed in the table of altered hearing tendencies in FIG. 2, altered hearing of "r" and a nasal sound, such as ┌ 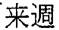 (RAISHUU)┘ and ┌ 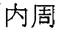 (NAISHUU)J is displayed in the middle row and in the lower row as words that are likely to be misheard by a person who has a moderate degree or a severe degree of weakened hearing ability. Therefore, in this case, determination in this hearing test pattern yields a determination result of "possibly having a moderate degree of impaired hearing ability or higher". That is, the determination result indicates the possibility of having a moderate degree of impaired hearing ability and also indicates the possibility of having a severe degree thereof.

Therefore, in order to clarify the determination result, in the same manner as the case of 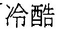 ┌ (REIKOKU)┘ (step S404), the second question sentence is next presented to the respondent, and the processes of steps S406 to S411 are performed.

When the respondent responds to the first question with ┌ 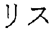 (RISU)┘ (step S415), the respondent does not mishear the word ┌ 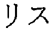 (RISU)J . However, as displayed in FIG. 2, ┌ 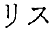 (RISU)J is classified as a word that is likely to be misheard by a person who has a severe degree of weakened hearing ability. Therefore, determination in this hearing test pattern yields a determination result of "not corresponding to a severe degree of weakness (possibly having a moderate degree or a mild degree of weakness)" (step S416).

When the respondent responds to the first question with a word other than the above (step S417), determination in this hearing test pattern yields a result of "not determined" (step S418).

These determination results are output to the respondent through the result output unit 6 (step S419). Storing the determination results on a database and the like is the same as that in the hearing test pattern illustrated in FIG. 3.

As described above, according to the hearing test device 1 and the hearing test method of the present embodiment, a question sentence that includes at least one of words which are likely to be misheard by a person who has decreased hearing ability is presented to a respondent by the question output unit 4 (e.g., output by voice sound by a speaker or read by a conductor). The specific content of a question sentence is arbitrarily set, but the form thereof takes the form of a questionnaire and the like. Thus, the respondent focuses on responding to the content of the question sentence that he/she listens to and is unlikely to sense that he/she receives a hearing test. Therefore, for example, it is possible to prompt the respondent who is confident that the hearing ability is not decreased to respond to the question sentence without making him/her be aware of taking a hearing test and without impairing the self-esteem of the respondent.

A determination of whether the hearing ability of the respondent is decreased is performed by the determining unit 12 on the basis of the content of a response that the respondent inputs, and the determination result is output to the respondent by the result output unit 6 (e.g., by displaying on a display screen or by printing out). As such, by presenting a determination result to the respondent from the determining unit 12, it is possible to provide the respondent who has decreased hearing ability with an opportunity to be aware of the degree of the decrease. Therefore, for example, it is possible to make the respondent who has decreased hearing ability realize the necessity of a hearing aid early and to prompt him/her to use a hearing aid before losing the language recognition ability thereof due to the progression of the decreased hearing ability.

A question sentence is provided in the form of a questionnaire or in the form of a quiz and the like. Thus, it is possible to determine the hearing ability of a respondent whose hearing ability is not decreased by allowing him/her to respond to the question with ease in a natural way without making him/her be aware of taking a hearing test. Therefore, for example, a hearing aid salesperson can efficiently sell hearing aids by questioning in advance people for whom hearing aids are necessary and people for whom hearing aids are not necessary.

Words that the subject having weakened hearing ability is likely to mishear are prepared in advance on the recording unit 2 so as to be linked to candidates of the words that are likely to be misheard. Thus, it is possible to widely cope with many possible mishearing patterns, and it is possible to accurately determine the level of the hearing ability of the respondent. In addition, it is possible to easily determine whether mishearing of the respondent is due to weakened hearing ability or is due to simple misunderstanding by determining whether the word with which the respondent responds is included in the set of words recorded on the recording unit 2 that are linked to with each other.

The level of decreased hearing ability can be determined depending on how the respondent mishears words, that is, not only depending on whether the response is "correct" or "incorrect" but also by reviewing the content of incorrectness (mishearing). Thus, it is possible to determine the level of the hearing ability of the respondent more accurately. In addition, question sentences each of which includes words having different possibilities of being misheard are prepared, and the plurality of question sentences is presented in stages in order to the respondent during the questionnaire and the like. Thus, it is possible to finely and accurately determine the level of the decreased hearing ability of the respondent on the basis of altered hearing tendencies depending on the degree of decreased hearing ability displayed in FIG. 2.

Frequency information that indicates the frequency of the use of the corresponding word is stored by being attached to each word stored on the recording unit 2. Thus, the question creating unit 11 can create a question sentence with a plurality of patterns by using the words that have the same frequency of use, and it is possible to prevent responses of the respondent from occurring of tendencies depending on certain words.

The voice sound that is output from the question output unit 4 has a different frequency in the case of a male voice sound and in the case of a female voice sound. In addition, people tend to have difficulty in listening as the frequency of sound is higher. Therefore, when the present embodiment is performed in the form of a questionnaire by a conductor as the question output unit 4, the determination criteria of the determining unit 12 may be adjusted depending on whether the questionnaire conductor is a male person or a female person. For example, when a female person conducts the questionnaire, the level of decreased hearing ability may be adjusted to a level that is one rank higher than that in the case of a male person. The determination criteria may be adjusted by applying a frequency filter to the voice sound output and making the high-frequency sound difficult to be heard by decreasing the volume thereof.

When a question sentence is output by voice sound, it is considered that the content of the question is estimated and determined from the intonation of the voice sound. Thus, it is desirable that the voice sound output is performed in a flat pronunciation as possible.

The fact that a hearing test has been conducted may not be notified to the respondent who is determined not to have weakened hearing ability in consequence of the questionnaire (hearing test) by not outputting the result of the hearing test but outputting a unique question result that can be deduced from the questionnaire, that is, a result that is not related to the hearing test.

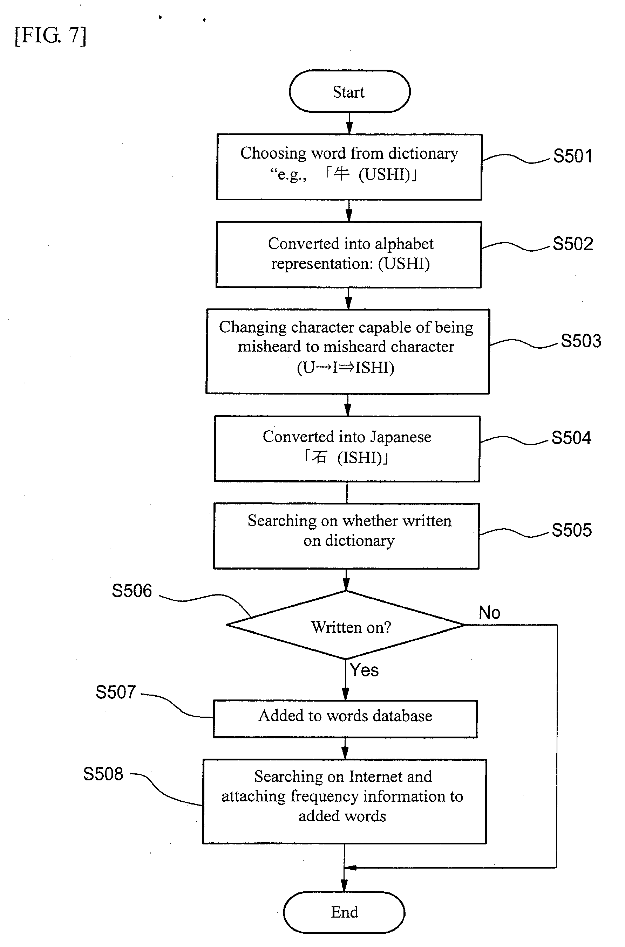

Next, an example of a method for creating the hearing test words in FIG. 2 recorded on the recording unit 2 will be described on the basis of the flowchart illustrated in FIG. 7.

First, a dictionary is prepared, and a word published on the dictionary is arbitrarily chosen (step S501, an example of a word selecting step). The case where the word chosen from the dictionary is ┌ 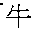 (USHI)┘ will be described below.

The chosen word (┌ 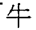 (USHI)┘ ) is converted into an alphabet representation (step S502, a Roman letter converting step).The alphabet representation of ┌ 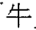 ┘ is "USHI".

Next, on the basis of the altered hearing matrix displayed in FIG. 2, a character (alphabet) of "USHI" that is capable of being misheard is changed to a misheard character (alphabet) (step S503, an alphabet changing step). A part "U" of "USHI" is changed to "I", and the whole is converted into "ISHI".

The "ISHI" that is created by the change is converted into a Japanese representation by using, for example, an electronic dictionary, a personal computer or the like (step S504). In this description, the case of being converted into ┌ 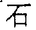 (ISHI)┘ will be described (steps S502 to S504 are examples of an altered hearing converting step).

A search is performed to find out whether the Japanese representation ┌ 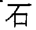 (ISHI)┘ exists as a word, that is, whether it is written on the dictionary (step S505, an example of a word searching step). A determination of whether it is written on the dictionary is made from the search result (step S506), and when being written, the word is additionally recorded on the recording unit 2 (step S507, an example of a word recording step). At the time of the recording, the word ┌ 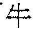 (USHI)J that is capable of being misheard is recorded so as to be linked to the candidate word ┌ 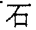 (ISHI)┘ of the mishearing thereof (refer to FIG. 2). The number of candidate words that are recorded so as to be linked thereto may be two or more.

The words ┌ 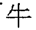 (USHI)┘ and ┌ 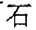 (ISHI)┘ at are to be additionally recorded, for example, are searched on the Internet, and the numbers of hits thereof, that is, the possibilities of using the words in an actual daily life are recorded as frequency information attached to the words (step S508, refer to FIG. 2). In FIG. 2, both the numbers of hits of ┌ 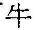 (USHI)┘ and ┌ 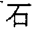 (ISHI)┘ are recorded as 500. When the number of candidate words of mishearing is two or more, an access may be made to various databases such as a literature database, a patent database and a thesis database connected to a network and information recorded on each database are compared with each other and checked, and as a result, the word having the greatest number of hits may be preferentially recorded on the recording unit 2. Alternatively, a degree of commonly-known may be set as a reference for a comprehensive determination by considering not only the number of hits but also a degree of recognition of the word (e.g., in the case of a kanji word, whether a commonly used kanji word or not) with respect to general consumers, and the degree of commonly-known may be recorded. While the altered hearing matrix displayed in FIG. 2 provides the example in which the frequency information (number of hits) is recorded for each word, the above-mentioned degree of commonly-known may be recorded.

As described above, according to the hearing test word creating method of the present embodiment, since a word that may be actually used (word that is published on a dictionary) is searched and recorded at the time of recording a misheard word on the recording unit 2 of the hearing test device 1, it is possible to widely cope with various responses of a respondent, and it is possible to accurately determine the level of hearing ability.

In addition, since the number of hits in an Internet search is referred to at the time of recording a misheard word on the recording unit 2 of the hearing test device 1, it is possible to record a word that has a high possibility of being used actually, and it is possible to determine the level of hearing ability more accurately.

While the present invention is described in detail with reference to a certain embodiment, it is apparent for those skilled in the art that various modifications and corrections can be added thereto without departing from the spirit and the scope of the present invention.

The present application is based upon Japanese Patent Application No. 2013-054053, filed on March 15, 2013, the content of which is incorporated herein by reference.

The present invention is not limited to Japanese and English and is able to be applied to other languages in the same manner. For example, in the case of applying the present invention to English, the altered hearing matrices disclosed in "The Journal of the Acoustical Society of America Vol. 128 (2010), pp. 444-455" and the like may be used. Specifically, examples of a set of English words that may be misheard include "Deer" and "Tear", "Sheep" and "Cheap", "Peach" and "Beach", and "Seed" and "Seat".

### DESCRIPTION OF REFERENCE NUMERALS AND SIGNS

- 1:: Hearing test device
- 2:: Recording unit
- 3:: Control unit
- 4:: Question output unit
- 5:: Input unit
- 6:: Result output unit

- 10:: CPU
- 11:: Question creating unit
- 12:: Determining unit

## Claims

1. A hearing test device comprising:
a recording unit on which a plurality of words having possibilities of being misheard are recorded in a state of being classified on the basis of highness and lowness of the possibilities;
a question output unit that outputs a question sentence containing at least one of the words by voice sound;
an input unit that receives input of a response to the question sentence;
a determining unit that determines at least whether the hearing ability of a respondent is decreased or not on the basis of the content of the response input into the input unit; and
a result output unit that outputs a determination result determined by the determining unit.

2. The hearing test device according to Claim 1,
wherein on the recording unit, the word having a possibility of being misheard is recorded in a state of being linked to a candidate word of the mishearing thereof.

3. The hearing test device according to Claim 1,
wherein the question output unit outputs in order a plurality of question sentences that include the words having a different possibility of being misheard on the basis of the content of the response that is input into the input unit, and
the determining unit determines a level of decreased hearing ability on the basis of the contents of the responses to the plurality of question sentences.

4. A hearing test method comprising:
a question outputting step of outputting by voice sound a question sentence that includes at least one word selected from a plurality of words having different possibilities of being misheard;
an inputting step of receiving input of a response to the question sentence;
a determining step of determining at least whether the hearing ability of a respondent is decreased or not on the basis of the content of the response that is input through the inputting step; and
a result outputting step of outputting a determination result determined through the determining step.

5. A hearing test word creating method that is a word creating method for creating a word which is recorded on a recording unit of the hearing test device according to Claim 1, the hearing test word creating method comprising:
a word selecting step of selecting a word arbitrarily from a dictionary;
an altered hearing converting step of performing an altered hearing conversion on the word that is selected in the word selecting step;
a word searching step of searching for whether a word resulted from the altered hearing conversion in the altered hearing converting step exists on the dictionary; and
a word recording step of, when the word gets a hit as an existing word in the word searching step, recording the word on the recording unit.

6. The hearing test word creating method according to Claim 5,
wherein the altered hearing converting step is a process of replacing a part of the characters of the word that is selected in the word selecting step on the basis of an altered hearing matrix that indicates altered hearing tendencies of a predetermined word group.

7. The hearing test word creating method according to Claim 5 or 6,
wherein when there are a plurality of words that get hits as existing words in the word searching step, they are compared with information recorded in a database on a network, and in consequence of the comparison, a word having a high frequency of use is recorded on the recording unit in the word recording step.
